# EUROPEAN PATENT SPECIFICATION

(11) **EP 2 521 912 B1**
(45) Date of publication and mention of the grant of the patent: **12.10.2016**
(21) Application number: 10800895.4
(22) Date of filing: 30.12.2010
(51) Int. Cl.: G01N 33/487, G01N 33/49, G01N 27/22

(54) **SAMPLE CHARACTERIZATION BASED ON AC MEASUREMENT METHODS**
PROBENCHARAKTERISIERUNG AUF DER BASIS VON AC-MESSVERFAHREN
CARACTÉRISATION D'ÉCHANTILLON BASÉE SUR LES PROCÉDÉS DE MESURE AC

(30) Priority: 08.01.2010 US 684277
(43) Date of publication of application: 14.11.2012
(73) Proprietor: Roche Diabetes Care GmbH, 68305 Mannheim (DE); F.Hoffmann-La Roche AG, 4070 Basel (CH)
(72) Inventor: LICA, Georgeta, Indianapolis, IN 46202 (US); BUCK, Harvey, B., Indianapolis, IN 46256 (US); GROLL, Henning, Tucson, AZ 85737 (US)
(86) International application number: PCT/EP2010/007990
(87) International publication number: WO 2011/082820

(56) References cited:
- WO-A2-2004/113896
- US-A1- 2004 157 337
- US-A1- 2004 256 248
- US-A1- 2008 202 927

## Description

### BACKGROUND

Diabetic therapy typically involves two types of insulin treatment: basal and meal-time. Basal insulin refers to continuous, e.g. time-released, insulin often taken before bed. Meal-time insulin treatment provides additional doses of faster acting insulin to regulate fluctuations in blood glucose caused by a variety of factors, including the metabolization of sugars and carbohydrates. Proper regulation of blood glucose fluctuations requires accurate measurement of the concentration of glucose in the blood. Failure to do so can produce extreme complications, including blindness and loss of circulation in the extremities, which can ultimately deprive the diabetic of use of his or her fingers, hands, feet, etc.

Multiple methods are known for measuring the concentration of analytes in a blood sample, such as, for example, glucose. Such methods typically fall into one of two categories: optical methods and electrochemical methods. Optical methods generally involve reflectance or absorbance spectroscopy to observe the spectrum shift in a reagent. Such shifts are caused by a chemical reaction that produces a color change indicative of the concentration of the analyte.

Conventional electrochemical methods generally include applying a constant potential or a potential step necessary to initiate the reaction of interest and measuring the resulting charge or current proportional to the glucose concentration. See, for example, U.S. Patent Nos. 4,233,029 to Columbus; 4,225,410 to Pace; 4,323,536 to Columbus; 4,008,448 to Muggli; 4,654,197 to Lilja et al.; 5,108,564 to Szuminsky et al.; 5,120,420 to Nankai et al.; 5,128,015 to Szuminsky et al.; 5,243,516 to White; 5,437,999 to Diebold et al.; 5,288,636 to Pollmann et al.; 5,628,890 to Carter et al.; 5,682,884 to Hill et al.; 5,727,548 to Hill et al.; 5,997,817 to Crismore et al.; 6,004,441 to Fujiwara et al.; 4,919,770 to Priedel et al.; and 6,054,039 to Shieh. According to the Cotrell equation, besides the concentration of the analyte of interest, factors such as temperature, electrode surface area, and diffusion coefficient also contribute to the resulting current. As a consequence, any variance in these parameters caused by changes in the temperature in the reaction zone, blockage of the electrode surface area, or impediments of reactant diffusion to the electrode surface would also affect the resulting current. Moreover, interfering compounds that are also electrochemically active at the applied potential can generate additional DC current and consequently, positively- biased glucose concentration.

Some of these issues have been avoided in the past by using AC impedance to obtain a measure of the contribution of the factors described above and further correct the DC current for an accurate glucose concentration estimation. These AC-based methods consist in the application of an alternative potential of variable frequency and the measurement of cell impedance. See, for example, U.S. Patent No. 6,797,150 to Kermani et al., U.S. Patent Application Publication No. 2004/0079652 to Vreeke et al., and European Patent No. 1 639 355 to Roche Diagnostics GmbH. However, these methodologies extend the measurement time due to their sequential application of AC and DC signals and the steady-state nature of the DC current.

Thus, there is a need for improvement in this field.

### SUMMARY

One aspect concerns a technique for detecting analyte concentrations according to claim 1.

Further forms, objects, features, aspects, benefits, advantages, and embodiments of the present invention will become apparent from a detailed description and drawings provided herewith.

### BRIEF DESCRIPTION OF THE DRAWINGS

FIG. 1 graphically illustrates a dual mediating system according to one embodiment of the present disclosure.
FIG. 2a is a Nyquist plot of Zre versus Zim of the AC response at frequencies of 20480, 10240, 2048, 1024, 512, 256, and 128 Hz according to one embodiment of the present disclosure.
FIG. 2b is a Nyquist plot of Zre versus Zim of the AC response at frequencies of 20000, 10000, 2000, 1000, 512, and 128 Hz for two different glucose concentrations at two different excitation modalities according to one embodiment of the present disclosure.
FIG. 3a is a plot of impedance versus time over a five second measurement time at 1024 Hz in which the test strip was dosed with blood solutions containing a glucose concentration of 150 mg/dl according to one embodiment of the present disclosure.
FIG. 3b is a plot of impedance versus time over a five second measurement time at 1024 Hz in which the test strip was dosed with blood solutions containing a glucose concentration of 120 mg/dl according to one embodiment of the present disclosure.
FIG. 4 graphically illustrates a sequence of reactions for a dual mediating system following an applied AC excitation signal according to one embodiment of the present disclosure.
FIG. 5 is a Nyquist plot of Zre versus Zim of the AC response at frequencies of 10000, 1000, 562, 316, 177, 100, 46, 21, 10, and 8 Hz at various glucose concentrations according to one embodiment of the present disclosure.
FIG. 6 are Bode plots of Zre and Zim versus frequency of the AC response at frequencies of 10000, 1000, 562, 316, 177, 100, 46, 21, 10, and 8 Hz at various glucose concentrations according to one embodiment of the present disclosure.
FIG. 7 is a Nyquist plot of Zre versus Zim of the AC response according to one embodiment of the present disclosure.
FIG. 8 is a block diagram of an equivalent circuit model used to fit the impedance data according to one embodiment of the present disclosure.
FIG. 9 graphically illustrates the equivalent circuit values obtained by fitting the impedance data with the equivalent circuit model shown in FIG. 8.
FIG. 10 is a Nyquist plot of Zre versus Zim of the AC response according to one embodiment of the present disclosure.
FIG. 11 graphically illustrates the equivalent circuit values obtained by fitting the impedance data with the equivalent circuit model shown in FIG. 8.
FIG. 12 graphically illustrates a sequence of reactions for a single mediating system following an applied AC excitation signal according to one embodiment of the present disclosure.
FIG. 13 is a Nyquist plot of Zre versus Zim of the AC response according to one embodiment of the present disclosure.

### DESCRIPTION OF THE SELECTED EMBODIMENTS

For the purpose of promoting an understanding of the principles of the invention, reference will now be made to the embodiments illustrated in the drawings and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one skilled in the art to which the invention relates. In particular, although the invention is discussed in terms of a blood glucose measurement method, it is contemplated that the invention measure other analytes and other sample types. Such alternative embodiments require certain adaptations to the embodiments discussed herein that would be obvious to those skilled in the art.

As will be described in detail below, a low amplitude AC waveform is applied to a body fluid sample in a biosensor and the impedance magnitude, phase angle, real or imaginary impedance, or other resistance and capacitance terms that correlate to the electrochemical cell properties or the biosensor are measured. The choice of the applied waveform may be determined by the time constant of the process of interest (i.e., fast or slow processes). The measured observables (phase, angle, magnitude, real or imaginary impedance) can then be converted to resistance and constant phase elements by fitting their values with an equivalent circuit and further used to provide an estimate of the sample glucose concentration, hematocrit, and/or temperature. In one example, the biosensor includes a mediator system that generates a linear faradic response at relatively low applied potential differences. The response at lower frequencies of the applied AC waveform is used to measure glucose concentration, and the response at the higher frequencies is used to detect the effects of temperature and hematocrit.

According to the invention, a high frequency AC signal (that is sensitive to the sample hematocrit and/or temperature) is applied for a given period until no significant change in the system impedance is observed, after which a second low frequency AC signal is then applied to determine the glucose concentration.

The impedance data can be processed by fitting it with an equivalent circuit model. The choice of the equivalent circuit is determined by the quality of the fit to the experimental data. This facilitates the accurate measurement of an analyte in a fluid. In particular, the measurement of the analyte remains accurate despite the presence of interferants, which would otherwise cause error. For example, with this technique, the concentration of blood glucose is measured without error that is typically caused by variations in the hematocrit or sample temperature. The accurate measurement of blood glucose is invaluable to the prevention of blindness, loss of circulation, and other complications of inadequate regulation of blood glucose in diabetics. This technique allows measurements to be made more rapidly and with less complex instrumentation, making it more convenient for the diabetic person to measure their blood glucose. Likewise, accurate and rapid measurement of other analytes in blood, urine, or other biological fluids provides for improved diagnosis and treatment of a wide range of medical conditions.

In the context of systems for measuring glucose, it will be appreciated that electrochemical blood glucose meters typically (but not always) measure the electrochemical response of a blood sample in the presence of a reagent. The reagent reacts with the glucose to produce charge carriers that are not otherwise present in blood. Consequently, the electrochemical response of the blood in the presence of a given signal is intended to be primarily dependent upon the concentration of blood glucose. Secondarily, however, the electrochemical response of the blood to a given signal is dependent upon other factors, including hematocrit and temperature. See, for example, U.S. Patents Nos. 5,243,516; 5,288,636; 5,352,351; 5,385,846; 5,508,171, and 6,645,368 which discuss the confounding effects of hematocrit on the measurement of blood glucose. In addition, certain other chemicals can influence the transfer of charge carriers through a blood sample, including, for example, uric acid, bilirubin, and oxygen, thereby causing error in the measurement of glucose.

The various embodiments disclosed herein relate to methods that allow shorter test times to be achieved while still delivering an analyte measurement (be it blood glucose or another fluid sample analyte) corrected for confounding interferents (be they hematocrit and temperature or other interferents). As used herein, "test time" is defined as the time from sample detection (or sample dose sufficiency, if both are detected) when a first electrical signal is to be applied to the sample to the taking of the last measurement used in the concentration determination calculation. As used herein, a low potential AC excitation refers to an applied AC potential difference between a working electrode and a counter electrode that is sufficient to generate a linear response. In one embodiment, a dual mediator system is utilized. In combination with a dual mediator system and glucose specific chemistry, the disclosed method can provide a complete spectrum of the sample investigated. Alternatively, a single mediator system may be utilized.

In selected experiments, which will be described below, measurements were conducted with an electrochemical test stand constructed on the basis of VXI components from Agilent, and programmable to apply AC potentials to sensors in requested combinations and sequences and to measure the resulting current responses of the sensors. A convenient sensor layout can be represented by the electrochemical ACCU-CHEK® AVIVA^{™} blood glucose strip, which includes gold working and counter electrodes coated with glucose specific chemistry. In certain embodiments, these chemical reagents include an enzyme and a mediator capable of specifically oxidizing the glucose and facilitating the electron transfer to the gold electrodes.

During the experiments, data was transferred from the electrochemical analyzer to a desktop computer for analysis using Microsoft® Excel®. The measurements in other examples can be carried out by any commercially available programmable potentiostat with an appropriate frequency response analyzer and digital signal acquisition system. For example, Gamry and CH Instruments potentiostats or multi-channel fast test stands may be used. For commercial use, the method can be carried out in a dedicated low-cost hand-held measurement device, such as the ACCU-CHEK® AVIVA^{™} blood glucose meter. In such a case, the measurement parameters may be contained in and/or provided to the firmware of the meter and the measurement sequence and data evaluation executed automatically with no user interaction.

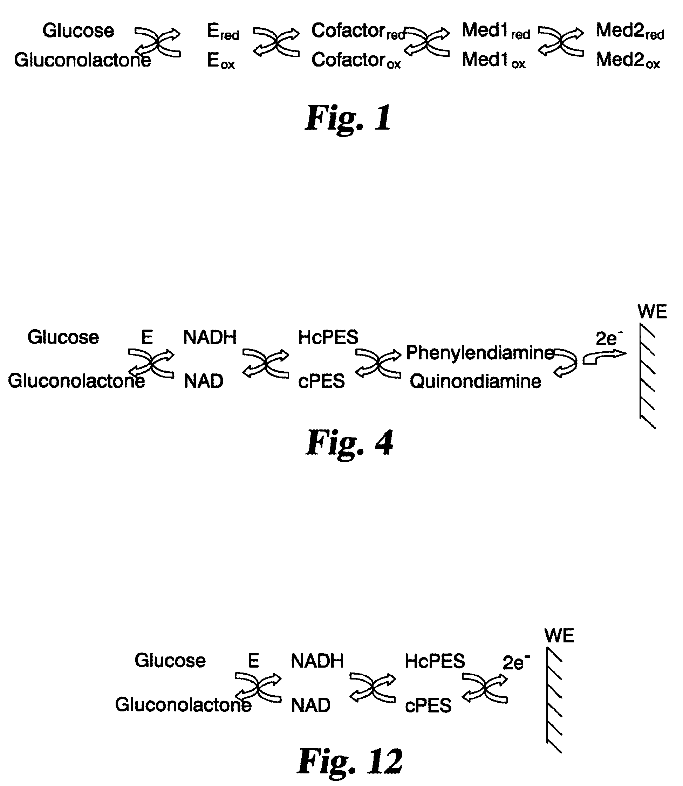

In selected embodiments, the mediator system includes an electron shuttle/mediator pair, cofactor/mediator pair, or mediatorl/mediator2 pair or other combination of the thereof. In the presence of glucose, the reaction sequence can be described by the scheme illustrated in FIG. 1.

In FIG. 1, E_{red} and Eₒₓ represent the reduced or oxidized forms of the enzyme, selected from a family of enzymes that specifically oxidize glucose (e.g., GDH). Cofactor_{red} and Cofactorₒₓ represent the reduced or oxidized forms of the cofactor, which is capable of facilitating the electron transfer from the enzyme active center to a mediator system (e.g., NAD/NADH). Med1_{red}, Med1_{od}, Med2_{red}, and Med2ₒₓ represent the reduced or oxidized forms of the mediators, selected from a family of compounds capable of facilitating the electron transfer to the electrodes to another mediator system and of being regenerated under applied potential and to participate in fast reversible redox reactions (e.g., phenazine, quinones, ferricyanide, transitional metal complexes). By using the chemistry shown in FIG. 1, the glucose concentration can be measured in a liquid sample upon the application of sufficient amount of sample to fill the capillary of the electrochemical strip.

The testing included dosing the strip with a glucose solution (aqueous, blood, serum). Once the sample was detected, an excitation signal including an AC potential of low amplitude potential difference (12 mV RMS or other) was applied to the working electrode of the sensor.

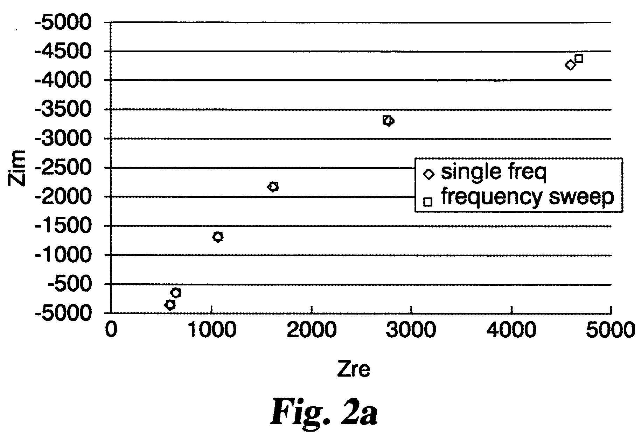

FIG. 2a is a Nyquist plot obtained by dosing the ACCU-CHEK® AVIVA^{™} blood glucose test strips with control solutions containing 572 mg/dl glucose. The applied AC frequencies were as follows: 20480, 10240, 2048, 1024, 512, 256, and 128 Hz. The diamond shaped points in FIG. 2a were obtained by applying the AC signal as a single frequency and reconstituting the spectrum from individual measurements by analyzing the data at 3 seconds into the test. The square shaped points were obtained by applying the AC signal as a first frequency sweep from 20480 to 1024 Hz, followed by a second block containing the frequencies 1024 to 128 Hz.

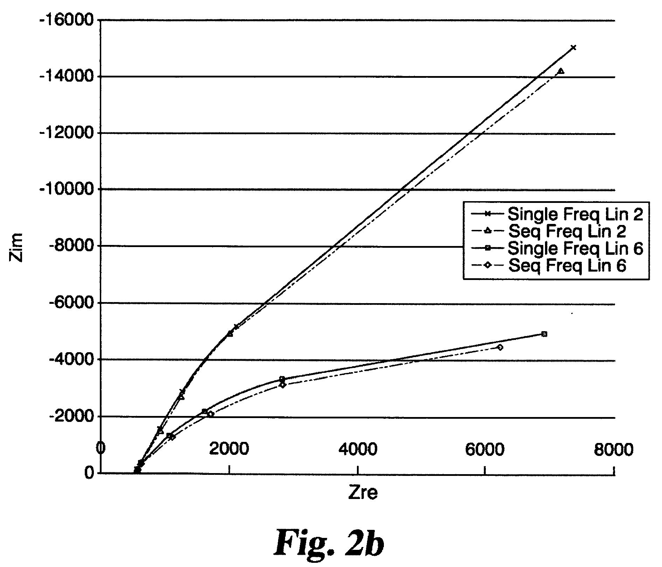

FIG. 2b is a Nyquist plot obtained by dosing the ACCU-CHEK® AVIVA^{™} blood glucose test strips with control solutions containing 57 and 572 mg/dl glucose. Line 2 ("Lin 2") in FIG. 2b represents the response for the 57 mg/dl glucose solution, and Line 6 ("Lin 6") represents the response for a 572 mg/dl solution. The applied AC frequencies were as follows: 20000, 10000, 2000, 1000, 512, and 128 Hz, and for the measurements, the reagent included cPES/NA chemistry. A comparison of the impedance response obtained using a single frequency or a multi-frequency sequence is shown in FIG. 2b. As can be seen, it was discovered that the responses were generally equivalent, either using a single frequency or multiple frequencies. Consequently, it was found that the measurement time can be reduced by applying all of the frequencies simultaneously.

It should be noted that there are different ways in which the system response may be represented, such as impedance in time, Nyquist plots, and Bode plots. A Nyquist plot, such as FIGS. 2a and 2b, displays the real and imaginary impedance components (Zre vs. Zim) at different frequencies. The shape of the graph provides information specific to the reaction mechanism, solution or electrode resistance, and reaction kinetics. However, one issue with this type of representation is that it does not contain explicit frequency information. This information is more evident in the Bode plots, where the impedance magnitude or phase angle is plotted versus the logarithm of frequency.

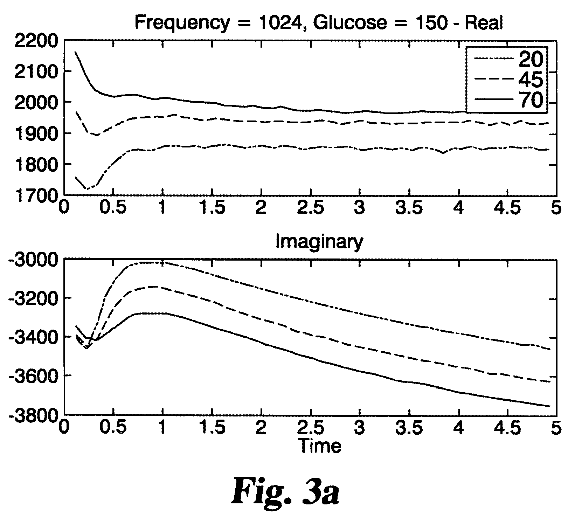

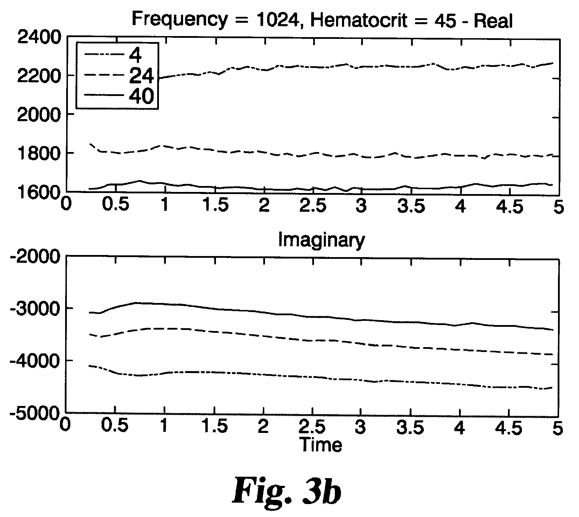

To achieve accurate measurements, the evolution of the measurement system over time needs be understood. It is helpful to appreciate when stable conditions occur so as to know when accurate measurements can be made. For instance, it is useful to understand when system hydration film swelling ceases. As mentioned before, the technique described herein measures glucose levels with low-frequency AC signals, and it measures the effects of temperature and hematocrit with high-frequency AC signals. FIG. 3a generally illustrates the evolution overtime in relation to measuring hematocrit, and FIG. 3b generally depicts this evolution in relation to temperature. Specifically, FIGS. 3a and 3b plot the AC impedance response over a 5 second measurement time at 1024 Hz. In FIG. 3a, the ACCU-CHEK® AVIVA^{™} blood glucose test strips were dosed with blood solutions containing 150 mg/dl glucose with 20,45, and 70% hematocrit levels. For FIG. 3b, the ACCU-CHEK® AVIVA^{™} blood glucose test strips were dosed with blood solutions containing 120 mg/dl glucose and nominal hematocrit after equilibrating for 1 minute at 4, 24, and 40°C. According to the invention, a high-frequency AC signal, which is sensitive to hematocrit and/or temperature, is initially applied until there is no significant change in the system impedance. Afterwards, a second, low-frequency AC excitation signal is applied. The response to this excitation low-frequency AC signal is indicative of the glucose concentration of the sample. In one example, the impedance data is further processed by fitting with an equivalent circuit model. The choice of the equivalent circuit is determined by the quality of the fit of the experimental data. Further, the type and sequence of elements in the circuit are associated with the sample physical properties and the processes occurring during the measurement.

The examples described below contain some specific cases where a measurement based only on AC excitation is utilized to determine the glucose concentration in aqueous and biological samples using different mediator systems, but do not constitute embodiments of the claimed invention.

### Example 1: Measurement of the glucose concentration in aqueous solutions

The goal of Example 1 was to identify the relevant frequency range for the diagnosis of glucose in a liquid sample. The glucose test strips utilized for this example contained a dual mediator system which reacts according to the scheme illustrated in FIG. 1 and detailed for this particular example the scheme illustrated in FIG. 4. The first mediator is a substituted phenazine (cPES) capable of accepting electrons from the enzyme cofactor (NAD). This mediator is also capable of participating in fast reversible reactions. As should be appreciated, this signifies that by applying only a low-potential AC excitation signal, one can achieve the conversion between the oxidized and reduced form of the mediator (cPES vs. HcPES), even on the time scales associated with high frequency measurements, and can generate a corresponding change in the cell impedance. The second mediator is nitrosoaniline (NA), which in the presence of the enzymatic reaction is converted to the quinonediimine form and capable of exchanging electrons with the gold electrodes under an applied potential.

Although Example 1 has been described with reference to a two-mediator type system, it should be recognized that less or more mediators can be used in other examples (e.g., one or three mediators). In general, the selected mediator(s) exhibit fast, reversible, low potential electron exchange. By eliminating NA, the chemistry matrix would be simplified and overall test strip costs would be reduced. However, the inclusion of NA to the mediating system assists in setting and maintaining the electrode potential in a bi-amperometric system, as well as expanding the glucose range that can be tested.

The testing performed for Example 1 included the following steps.

### Step 1: the electrochemical strip was dosed with a control solution containing 129 (lin 3), 524 (lin 5), and 1000 (lin 1000) mg/dl glucose.

Step 2: as soon as the sample was detected, a 12 mV RMS potential difference AC signal was applied between the working and counter electrodes on the strip. If the AC excitation was applied in the conventional way, as a sweep from high to low frequency, the measurement time necessary to cover the frequency range 20000 to 10 Hz for instance would be over 10 seconds. However, other processes, such as film hydration and swelling, occurred in this time frame and contributed to the impedance response. In order to isolate the enzymatic reaction from such time-dependent events, the AC signal was applied as a single frequency for a period of 5 seconds and a given glucose strip. The measurement was repeated for the desired number of frequencies in the selected frequency range. The Nyquist and Bode plots are then reconstituted by considering the impedance from individual measurements at a given time after the drop detect (for instance, 3 seconds).

### Step 3: under the applied AC input, the sequence of reactions illustrated in FIG. 4 took place.

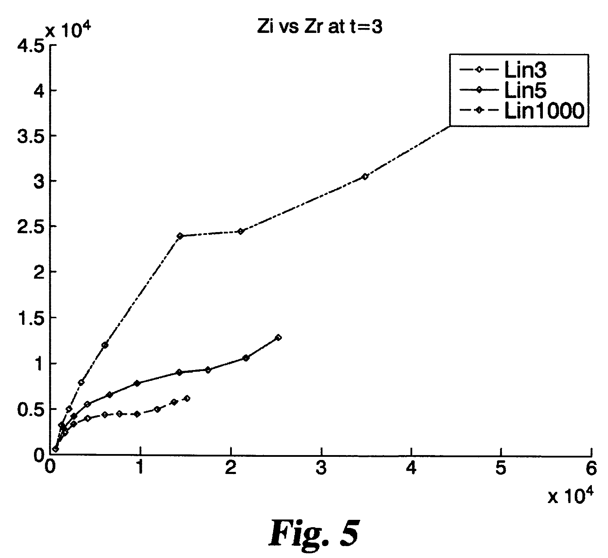

FIG. 5 illustrates Nyquist plots obtained for the cPES/NA chemistry when dosing with 129 (lin 3), 524 (lin 5), and 1000 (lin 1000) mg/dl glucose control solution at 3 seconds during the AC measurements. The applied AC excitation signal had the following frequencies: 10000, 1000, 562, 316, 177, 100, 46, 21, 10, and 8 Hz. Two distinct frequency ranges can be identified in the Nyquist plots of FIG. 5: 1) at high frequencies, the kinetic range consists of a open semicircle with the diameter being sensitive to the glucose concentration; and 2) at low frequencies, the diffusion range consists of a linear correlation of Zre and Zim.

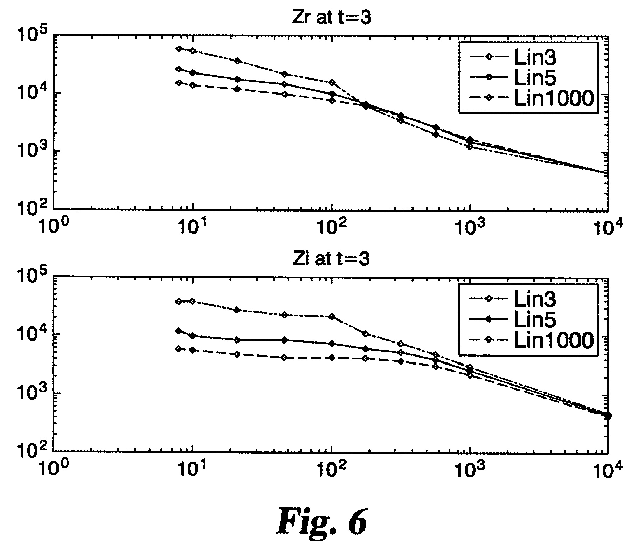

FIG. 6 are Bode plots of Zre and Zim versus frequency of the AC response obtained for the cPES/NA chemistry when dosing with 129 (lin 3), 524 (lin 5), and 1000 (lin 1000) mg/dl glucose control solution at 3 seconds during the AC measurements. The applied AC excitation signal had the following frequencies: 10000, 1000, 562, 316, 177, 100, 46, 21, 10, and 8 Hz. The Bode plots shown in FIG. 6 suggest that the relevant frequency range for the glucose detection is 1000 to 100 Hz. At frequencies higher than 1000 Hz, the impedance data is less sensitive to the glucose concentration. Also, at frequencies lower than 100 Hz, there is no improvement in the glucose dose response at the expense of increased measurement time.

### Example 2. Glucose detection in blood samples of variable temperature and hematocrit

Example 2 included applying a single low amplitude (12 mV RMS) potential difference AC excitation signal at different frequencies covering the 20000 to 100 Hz range for 5 seconds after the drop detect. For simplification, Example 2 was divided into 2 parts. The first part focused on investigating the glucose concentration and hematocrit effect. For the first part, 9 solutions were prepared using target glucose concentrations of 50, 150, and 500 mg/dl and target hematocrit values of 20, 45, and 70% adjusted at room temperature. The second part focused only on investigating the hematocrit and temperature effect. For the second part, 9 solutions were prepared each containing blood glucose concentration of 120mg/dl and hematocrit values of 20, 45, or 70%, measured after the strips equilibrated at 4, 24, or 40°C.

### Example 2.1: Glucose/Hematocrit Study

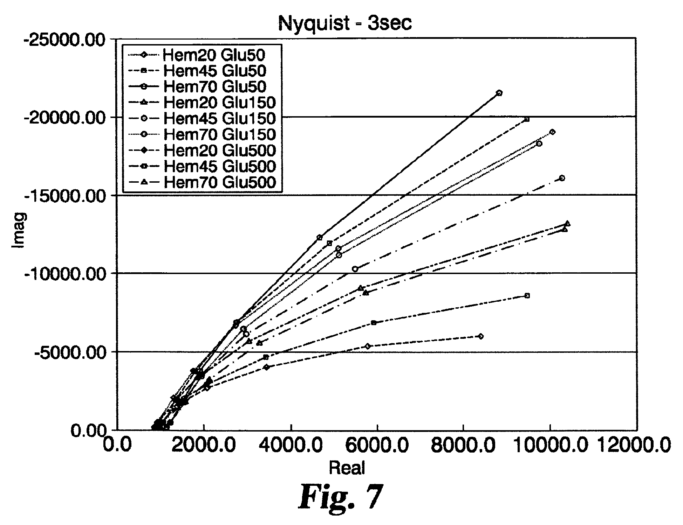

FIG. 7 includes nine Nyquist plots obtained for the cPES/NA chemistry when dosing with blood solutions containing 50, 150, and 500 mg/dl glucose and hematocrit adjusted to 20, 45, and 70% adjusted at room temperature. The Nyquist plots suggest that the impact of the hematocrit and glucose concentration is mostly along the Zre axis.

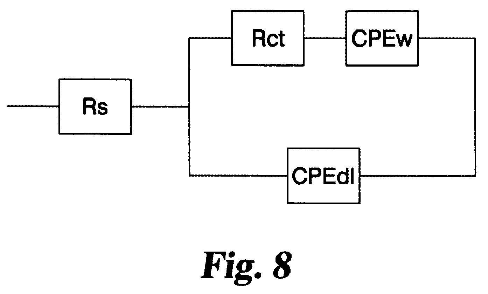

In order to obtain a better understanding of this response, the impedance data was further fitted with the equivalent circuit of FIG. 8. The elements in the equivalent circuit represent the following:
Rₛ: solution resistance
R_{ct}: resistance to charge transfer: represents a measure of the reaction kinetics: the faster the reaction or the higher the surface concentration, the smaller R_{ct}
CPE_{w}: diffusion capacitance
CPE_{dl}: capacitance of the double layer for a non-homogeneous interface

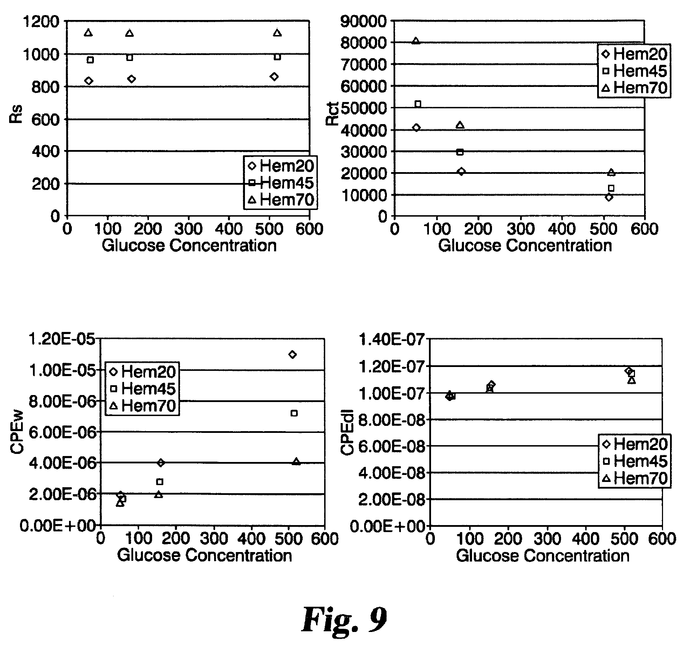

FIG. 9 graphically illustrates the equivalent circuit element values obtained by fitting the impedance data with the model shown in FIG. 8. The impedance data is fitted to the equivalent circuit based on known statistical methods (such as the chi-squared or sum of squares techniques as but two examples), as well as knowledge of the system being investigated. As noted above, the equivalent circuit makes the data easier to interpret and analyze. Additionally, the equivalent circuit elements contain information over a given frequency range. As a consequence, each element may be less affected by noise than a single frequency point.

The impedance data was determined from cPES/NA chemistry when dosing with blood solutions containing 50, 150, and 500 mg/dl glucose and hematocrit adjusted to 20, 45, and 70%. After analyzing FIG. 9, it becomes clear that hematocrit value impacts the resistance terms, manifesting an increased resistance with the blood cell content. At the same time, a high hematocrit acts as an impediment to the glucose diffusion, generating a reduced diffusion capacitance.

The glucose dose response can be seen along the R_{ct} and CPE_{w} elements, but it does not affect the Rₛ and C_{dl} terms. For this system, Rₛ provides a measure of the sample hematocrit, independent of the glucose concentration.

### Example 2.2: Hematocrit/Temperature Study

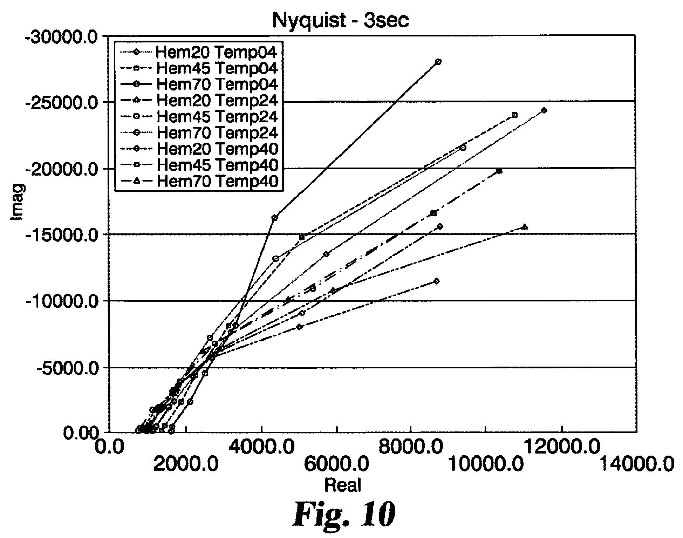

A similar analysis was performed for Example 2.2. FIG. 10 includes nine Nyquist plots obtained for the cPES/NA chemistry when dosing with blood solutions containing 120 mg/dl glucose and hematocrit adjusted to 20, 45, or 70% after equilibrating for 1 minute at 4, 24, or 40°C.

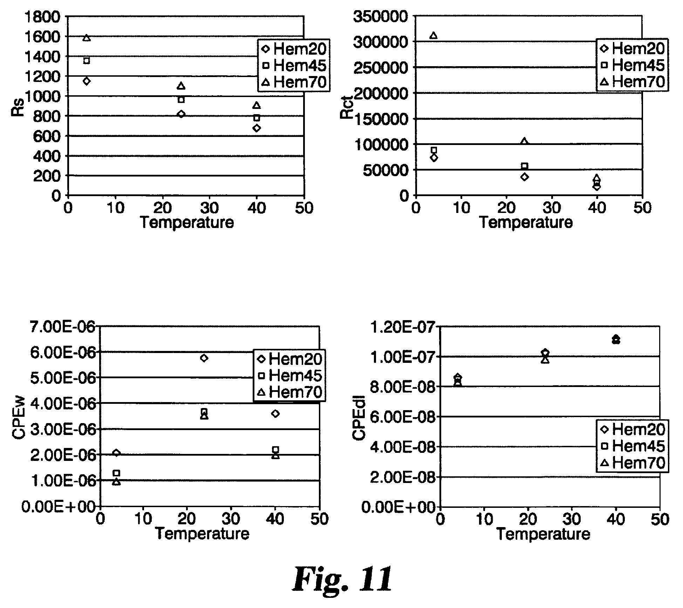

The impedance data shown in FIG. 10 was the fitted with the model as described above to determine the impact of temperature and hematocrit at constant glucose concentration on the equivalent circuit elements. The results of the algorithmic fit are shown in FIG. 11. Of note, the solution resistance (the glucose independent term) and the charge transfer resistance values were decreased with increased temperature, following a reversed trend compared to the hematocrit. The double-layer capacitance is sensitive to the change in the sample temperature. Therefore, it provides a measure of the temperature in the reaction zone, independent of hematocrit and glucose concentration.

### Example 3. Glucose detection with a new mediator system

The glucose detection presented in the above Examples was based on a specific enzymatic reaction coupled with two mediators that can further transfer the electrons to the electrode surface, as well as cause a change in the cell impedance proportional to the glucose concentration. The chemistry can be further simplified by eliminating one of the mediators. In this case, the reaction scheme shown in FIG. 4 will reduce to the scheme illustrated in FIG. 12.

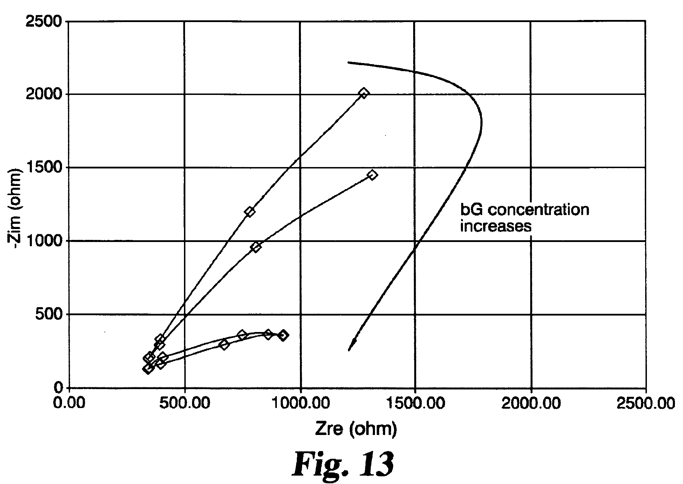

Under the applied AC signal, the change in the mediator oxidation state as a consequence of the enzymatic reaction can be monitored. FIG. 13 illustrates the Nyquist plots obtained for the cPES chemistry based strip when dosing with solutions containing no glucose, 57, 572, and 1200 mg/dl glucose concentrations. The frequencies tested were 20000, 10000, 2000, and 1000 Hz. The frequencies were applied as a sweep from high to low frequency, thereby reducing the measurement time to under 3 seconds. The results of Example 3 indicate that the glucose concentration impacts mostly the impedance at 2000 and 1000 Hz.

By the appropriate selection of the frequency of the applied signal, AC-based measurement can provide a complete spectrum of the sample to be analyzed.

The resulting sample response can then be measured and the contribution from each excitation frequency component can be deduced by use of Fourier Transform techniques, such as a Discrete Fourier Transform (DFT). Although the various examples disclosed herein utilize multi-sine excitation waveforms, those skilled in the art will recognize that the multi-frequency waveform may be constructed using individual waveforms having any desired shape, such as triangular, square, sawtooth, delta, etc., just to name a few nonlimiting examples. The component AC waveforms used to create the multi-frequency waveform may each have any desired frequency and any desired amplitude. The use of multi-frequency techniques not only shortens the time necessary to collect the desired data (since the AC measurements are made simultaneously rather than sequentially), but also correlates better for correction since the sample is varying less during the data collection corresponding to each applied frequency.

While the invention has been illustrated and described in detail in the drawings and foregoing description, the same is to be considered as illustrative and not restrictive in character, it being understood that only the preferred embodiment has been shown and described and that all changes, equivalents, and modifications that come within the scope of the following claims are desired to be protected.

## Claims

1. A method, comprising:
providing an electrochemical test strip with a mediator system that generates a linear faradic response at a low applied potential difference;
introducing blood to the mediator system and in contact with electrodes of the test strip;
applying an alternating current excitation signal as a potential difference between electrodes of the test strip and across the blood, wherein the alternating current excitation signal includes a low frequency signal and a high frequency signal that has a higher frequency than the low frequency signal; and
determining glucose concentration of the blood, wherein said determining glucose concentration includes
measuring a low frequency response to the low frequency signal,
measuring a high frequency response to the high frequency signal,
estimating the glucose concentration based on the low frequency response, and
correcting the glucose concentration for one or more error causing variables based on the high frequency response, **characterized in that** said determining the glucose concentration includes applying the high frequency signal until no significant change in impedance is observed in the high frequency response;
observing no significant change in the impedance of the high frequency response;
and
applying the low frequency signal after said observing no significant change in the impedance of the high frequency response.

2. The method of claim 1, wherein the alternating current excitation signal is selected from a range of 1 Hz to 20,000 Hz.

3. The method of any one of claims 1 to 2, wherein the alternating current excitation signal has a potential of at most 12 mV RMS.

4. The method of any one of claims 1 to 3, wherein the low frequency signal is at most 2000 Hz, preferably selected from a range of 1000 Hz to 2000 Hz or selected from a range of 100 Hz to 1000 Hz.

5. The method of any one of claims 1 to 4, wherein the high frequency signal is at least 2000 Hz.

6. The method of any one of claims 1 to 5, wherein said measuring the low frequency response and/or the high frequency response independently includes measuring phase angle, magnitude, resistance, capacitance, and/or impedance.

7. The method of any one of claims 1 to 6, wherein said determining the glucose concentration includes fitting the low frequency response and the high frequency response to an equivalent circuit.

8. The method of any one of claims 1 to 7, wherein the mediator includes a single mediator type system or a dual mediator type system.

## Patentansprüche

1. Verfahren, umfassend:
Bereitstellen eines elektrochemischen Teststreifens mit einem Mediatorsystem, das bei einem niedrigen angelegten Potentialunterschied eine lineare faradische Antwort erzeugt;
Einführen von Blut auf das Mediatorsystem und in Kontakt mit Elektroden des Teststreifens;
Anlegen eines Wechselstrom-Anregungssignals als Potentialunterschied zwischen Elektroden des Teststreifens und über das Blut, wobei das Wechselstrom-Anregungssignal ein niederfrequentes Signal und ein hochfrequentes Signal, das eine höhere Frequenz als das niederfrequente Signal aufweist, enthält; und
Bestimmen der Glucosekonzentration des Bluts, wobei das Bestimmen der Glucosekonzentration umfasst:
Messen einer niederfrequenten Antwort auf das niederfrequente Signal,
Messen einer hochfrequenten Antwort auf das hochfrequente Signal,
Abschätzen der Glucosekonzentration auf der Grundlage der niederfrequenten Antwort und
Korrigieren der Glucosekonzentration auf eine oder
mehrere fehlerverursachende Variablen auf der Grundlage der hochfrequenten Antwort, **dadurch gekennzeichnet, dass** das Bestimmen der Glucosekonzentration das Anlegen des hochfrequenten Signals, bis keine wesentliche Veränderung der Impedanz der hochfrequenten Antwort beobachtet wird, umfasst;
Beobachten keiner wesentlicher Veränderung der Impedanz der hochfrequenten Antwort;
und
Anlegen des niederfrequenten Signals nach der Beobachtung keiner wesentlicher Veränderung der Impedanz der hochfrequenten Antwort.

2. Verfahren gemäß Anspruch 1, wobei das Wechselstrom-Anregungssignal aus einem Bereich von 1 Hz bis 20.000 Hz ausgewählt ist.

3. Verfahren gemäß einem der Ansprüche 1 bis 2, wobei das Wechselstrom-Anregungssignal ein Potential von höchstens 12 mV RMS aufweist.

4. Verfahren gemäß einem der Ansprüche 1 bis 3, wobei das niederfrequente Signal bei höchstens 2000 Hz liegt, vorzugsweise ausgewählt aus dem Bereich von 1000 Hz bis 2000 Hz oder ausgewählt aus dem Bereich von 100 Hz bis 1000 Hz.

5. Verfahren gemäß einem der Ansprüche 1 bis 4, wobei das hochfrequente Signal bei wenigstens 2000 Hz liegt.

6. Verfahren gemäß einem der Ansprüche 1 bis 5, wobei das Messen der niederfrequenten Antwort und/oder der hochfrequenten Antwort unabhängig das Messen von Phasenwinkel, Höhe, Widerstand, Kapazität und/oder Impedanz umfasst.

7. Verfahren gemäß einem der Ansprüche 1 bis 6, wobei das Bestimmen der Glucosekonzentration das Anpassen der niederfrequenten Antwort und der hochfrequenten Antwort an einen äquivalenten Schaltkreis umfasst.

8. Verfahren gemäß einem der Ansprüche 1 bis 7, wobei der Mediator ein ein-Mediator-Typ-System oder ein zwei-Mediatoren-Typ-System umfasst.

## Revendications

1. Procédé, comprenant :
la fourniture d'une bandelette de test électrochimique avec un système de médiateur qui génère une réponse faradique linéaire à une faible différence de potentiel appliquée ;
l'introduction de sang dans le système de médiateur et en contact avec les électrodes de la bandelette de test ;
l'application d'un signal d'excitation en courant alternatif sous la forme d'une différence de potentiel entre les électrodes de la bandelette de test et à travers le sang, dans laquelle le signal d'excitation en courant alternatif comprend un signal basse fréquence et un signal haute fréquence qui a une fréquence supérieure à celle du signal basse fréquence ; et
la détermination de la concentration de glucose du sang, dans laquelle ladite détermination de la concentration de glucose comprend
la mesure d'une réponse en basse fréquence au signal basse fréquence,
la mesure d'une réponse en haute fréquence au signal haute fréquence,
l'estimation de la concentration de glucose sur la base de la réponse en basse fréquence, et
la correction de la concentration de glucose pour une ou plusieurs variables causant des erreurs sur la base de la réponse en haute fréquence, **caractérisée en ce que** ladite détermination de la concentration de glucose comprend l'application du signal haute fréquence jusqu'à ce que l'absence de modification significative de l'impédance soit observée dans la réponse en haute fréquence ;
l'observation de l'absence de modification significative de l'impédance de la réponse en haute fréquence ;
et
l'application du signal basse fréquence après ladite observation de l'absence de modification significative de l'impédance de la réponse en haute fréquence.

2. Procédé selon la revendication 1, dans lequel le signal d'excitation en courant alternatif est choisi dans une plage de 1 Hz à 20 000 Hz.

3. Procédé selon l'une quelconque des revendications 1 à 2, dans lequel le signal d'excitation en courant alternatif a un potentiel d'au plus 12 mV RMS.

4. Procédé selon l'une quelconque des revendications 1 à 3, dans lequel le signal basse fréquence est d'au plus 2 000 Hz, de préférence choisi dans une plage de 1 000 Hz à 2 000 Hz ou choisi dans une plage de 100 Hz à 1 000 Hz.

5. Procédé selon l'une quelconque des revendications 1 à 4, dans lequel le signal haute fréquence est d'au moins 2 000 Hz.

6. Procédé selon l'une quelconque des revendications 1 à 5, dans lequel ladite mesure de la réponse en basse fréquence et/ou la réponse en haute fréquence comprend de façon indépendante la mesure de l'angle de phase, de l'amplitude, de la résistance, de la capacitance et/ou de l'impédance.

7. Procédé selon l'une quelconque des revendications 1 à 6, dans lequel la détermination de la concentration de glucose comprend l'ajustement de la réponse en basse fréquence et la réponse en haute fréquence à un circuit équivalent.

8. Procédé selon l'une quelconque des revendications 1 à 7, dans lequel le médiateur comprend un système de type médiateur unique ou un système de type médiateur double.
